# EUROPEAN PATENT SPECIFICATION

(11) **EP 0 596 935 B1**
(45) Date of publication and mention of the grant of the patent: **18.10.1995**
(21) Application number: 92915980.4
(22) Date of filing: 20.07.1992
(51) Int. Cl.: A61K 9/00, A61K 9/20, A61K 31/52

(54) **ANTIVIRAL PHARMACEUTICAL COMPOSITIONS FOR VAGINAL ADMINISTRATION**
VIRUSHEMMENDE PHARMAZEUTISCHE ZUBEREITUNGEN ZUR VAGINALEN VERABREICHUNG
COMPOSITIONS PHARMACEUTIQUES ANTIVIRALES ADMINISTREES PAR VOIE VAGINALE

(30) Priority: 26.07.1991 IT MI912071
(43) Date of publication of application: 18.05.1994
(73) Proprietor: L.C. PHARCHEM LTD., Nicosia (CY)
(72) Inventor: CONTE, Ubaldo, I-21052 Busto Arsizio (IT); MAGGI, Lauretta, I-27100 Pavia (IT)
(74) Representative: Minoja, Fabrizio
(86) International application number: EP9201655
(87) International publication number: WO9302662

(56) References cited:
- EP-A- 0 020 777
- EP-A- 0 088 394
- EP-A- 0 219 161
- EP-A- 0 255 902
- BE-A- 658 905
- FR-A- 2 355 510
- GB-A- 2 199 495
- US-A- 4 983 393
- US-B- 4 389 393
- CHEMICAL ABSTRACTS, vol. 110, no. 19, 8 May 1989, Columbus, Ohio, US; abstract no. 165630h, HUSSAIN A.S. ET AL '"Body burden" of phosphonoformic acid after topical and vaginal administration to rabbits and beagle dogs'
- CHEMICAL ABSTRACTS, vol. 100, no. 13, 26 March 1984, Columbus, Ohio, US; abstract no. 96198c, KERN E.R. ET AL 'Acyclovir treatment of experimental genital herpes simplex virus infections.I. Topical therapy of type 2 and 1 infections of mice'
- CHEMICAL ABSTRACTS, vol. 114, no. 10, 11 March 1991, Columbus, Ohio, US;abstract no. 88498z, PARK Y. H. ET AL 'Preparation and evaluation of sustained release oral adhesive type acyclovir tablet'

## Description

The present invention refers to antiviral pharmaceutical compositions for vaginal administration.

Particular attention has been recently paid to the administration of drugs by the vaginal route in order to obtain, beside local effects, also systemic effects.

Usually, the drug is carried in form of vaginal ovules comprising semisynthetic glycerides (Remington's Pharmaceutical Sciences 17 Ex. p. 582) or natural fats (e.g. cocoa butter) having normally a melting or softening point at about 37°C, allowing the release of the drug for the absorption.

The drug may be solubilized in the fatty components or it may be homogeneously dispersed therein.

Other known forms for vaginal use include soft capsules, suited for non-hydrophilic, liquid drugs, oily dispersions or solutions, vaginal washes, ointments, gels.

Effervescent vaginal formulations of spermatocidal agents are disclosed in EP-A-88394.

The known compositions are not satisfactory since they cannot provide a sufficiently long permanence of the drug in contact with the vaginal mucosa.

Antiviral drugs are particularly suited for the vaginal administration.

The present invention provides effervescent tablets for vaginal administrations, containing acyclovir.

The sustained or prolonged release after vaginal administration may be obtained according to the invention by means of effervescent compositions.

The invention provides therefore antiviral vaginal tablets formulated so as to cause, when in contact with the liquids present in the application site, a slight, progressive and slow effervescence. The selection of the appropriate amounts of a organic and biocompatible acid and of an alkaline carbonate or bicarbonate will provide the desired effect.

For the preparation of tablets or other pharmaceutical forms for vaginal administration, excipients and technological additives suited to confer to the compositions the desired flowability and compactation characteristics as well as components useful to make the composition aesthetically acceptable, may also be used.

In order to evaluate the therapeutic characteristics of the compositions of the invention, clinical trials were carried out on vaginal effervescent tablets or slow-release bi-layered vaginal tablets containing 400 mg of acyclovir.

The results of the tests, carried out on 40 patients affected by relapsing Type 2 genital herpes treated with one tablet per day of Examples 1 or 2, have shown that the compositions of the invention are able to induce the regression of symptomatology more rapidly and with a better tolerability in comparison with the conventional vaginal formulations.

The invention is further illustrated by the following Examples.

### EXAMPLE 1

### Effervescent tablets containing acyclovir Unitary composition:

| | |
|---|---|
| acyclovir | 400.0 mg |
| lactose | 900.0 mg |
| maize starch | 242.0 mg |
| adipic acid | 140.0 mg |
| sodium bicarbonate | 110.0 mg |
| magnesium stearate | 20.0 mg |
| stearic acid | 8.0 mg |
| colloidal silica | 8.0 mg |
| polysorbate 80 | 2.0 mg |

### Preparation

A granulate containing the active principle is prepared by mixing acyclovir and maize starch together with an aqueous solution of starch paste and polysorbate 80.

The wet mass is forced through a screen (710 µ). The granulate is then dried to constant weight and sieved again.

Colloidal silica is added thereto and the mixture is mixed in a solid mixer for 10 minutes. Separately, a granulate containing adipic acid is prepared from lactose and maize starch. The two granulates are then mixed together in a powder mixer for 15 minutes. Sodium bicarbonate is then added and mixed for further 15 minutes. Stearic acid, magnesium stearate and colloidal silica (previously sieved) are finally added and mixed for further 20 minutes.

Tablets having ogival or almond shape and containing 400 mg of active principle are prepared from the obtained mixture.

### EXAMPLE 2

### Bi-layered vaginal tablets containing acyclovir

A first layer, effervescent, has the following unitary composition:

| | |
|---|---|
| acyclovir | 200.0 mg |
| lactose | 500.0 mg |
| maize starch | 122.0 mg |
| adipic acid | 70.0 mg |
| sodium bicarbonate | 55.0 mg |
| magnesium stearate | 10.0 mg |
| stearic acid | 4.0 mg |
| colloidal silica | 4.0 mg |
| polysorbate 80 | 1.0 mg |

The granulate is prepared according to the method of Example 1.

The second layer has the following unitary composition.

| | |
|---|---|
| acyclovir | 200 mg |
| hydroxypropylmethylcellulose (Methocel K 4 M) | 200 mg |
| mannitol | 400 mg |
| maize starch | 200 mg |
| adipic acid | 70 mg |
| talc | 20 mg |
| magnesium stearate | 10 mg |

The active principle, hydroxypropylmethylcellulose, mannitol, maize starch and adipic acid, previously sieved, are mixed for 20 minutes in a suitable mixer. The mixture is then added with magnesium stearate and talc and mixed for further 20 minutes.

Bi-layered tablets are prepared using a suitable tabletting machine (Kilian or Manesty) equipped with ogival punches and matrices.

The bi-layered tablet, automatically obtained, contains 200 mg of acyclovir in the first effervescent layer and 200 mg of acyclovir in the second layer consisting of hydrophilic, gelifiable and bioadhesive matrix, from which the active component is released in about 24 hours.

## Claims

1. Effervescent tablets for vaginal administration containing acyclovir.

## Patentansprüche

1. Aufbrausende Tabletten für Scheidenverabreichung, die Acyclovir enthalten.

## Revendications

1. Comprimés effervescents pour l'administration vaginale, contenant acyclovir.
